(19) Europäisches Patentamt
European Patent Office
Office européen des brevets

(11) Veröffentlichungsnummer: **0 039 889**
**B1**

(12) # EUROPÄISCHE PATENTSCHRIFT

(45) Veröffentlichungstag der Patentschrift: **27.07.83**

(51) Int. Cl.³: **C 07 C 33/02**, C 07 C 27/02, C 07 C 29/09, C 07 C 69/145

(21) Anmeldenummer: **81103408.1**

(22) Anmeldetag: **06.05.81**

(54) **Verfahren zur Herstellung von Amitinol.**

(30) Priorität: **12.05.80 DE 3018154**

(43) Veröffentlichungstag der Anmeldung: **18.11.81 Patentblatt 81/46**

(45) Bekanntmachung des Hinweises auf die Patenterteilung: **27.07.83 Patentblatt 83/30**

(84) Benannte Vertragsstaaten: **AT CH DE FR GB LI SE**

(56) Entgegenhaltungen:
**DE-B-1 125 910**
**US-A-3 433 839**
**AGRICULTURAL AND BIOLOGICAL CHEMISTRY, Tokyo, Band 38, September 1974.**
**KENJI MORI, »a new synthesis of 2-methyl 6-methyleneocta-2,7-dien-4-ol, a component of the pheromone of California five-spined«, Seiten 2045–2047.**

(73) Patentinhaber: **CELAMERCK GmbH & Co. KG, Binger Strasse 173, D-6507 Ingelheim am Rhein (DE)**

(72) Erfinder: **Mengel, Rudolf, Dr., Schützenpfad 22, D-6507 Ingelheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 039 889 B1

BUNDESDRUCKEREI BERLIN

Verfahren zur Herstellung von Amitinol

Die Erfindung betrifft ein Verfahren zur Herstellung von Amitinol, einem Aggregationspheromon des Borkenkäfers Ips amitinus [Naturwissenschaften 67, Seite 147—148 (1980)].

Amitinol hat die Formel

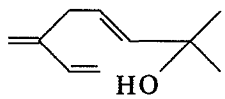

(I)

(E-2-Methyl-6-methylen-3,7-octadien-2-ol).

Es ist bekannt, Amitinol aus Ipsdienol herzustellen (DE-OS 2 750 604), indem man diesen Ausgangsstoff durch Behandlung mit Tetrafluorborwasserstoffsäure in das isomere Amitinol umlagert. Die Ausbeute dieses Verfahrens ist jedoch mäßig, da unter den Verfahrensbedingungen der entstehende tertiäre Alkohol z. T. erwartungsgemäß eine Wassereliminierung erleidet. Die Herstellung von Ipsdienol, etwa nach der o. g. DE-OS, ist ebenfalls nicht einfach.

Auch die Herstellung von Amitinol ausgehend von Myrcene ist bereits beschrieben worden (K. Mori, Agr. Biol. Chem. 38, 2045—2047 [1974]). Innerhalb dieses Verfahrens wird zunächst aus Myrcene ein Epoxid hergestellt und dieses dann mit dem sehr giftigen Phenylselenid, das in situ aus dem feuchtigkeitsempfindlichen und teuren Diphenylselenid erzeugt wird, weiter umgesetzt.

Gemäß der Erfindung wird der Weg über das Myrcene-Epoxid vermieden, so daß die mit dem bekannten Weg verbundenen Nachteile entfallen, ohne daß Qualität und Ausbeute des Endprodukts beeinträchtigt werden.

Gegenstand der Erfindung ist nun ein Verfahren, das von einem gut zugänglichen Ausgangsstoff auf einfache Weise und mit guter Ausbeute zum Amitinol führt.

Das erfindungsgemäße Verfahren geht von Myrcene

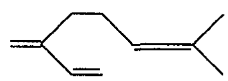

(II)

aus, an welches ein Sulfenylchlorid der Formel

$R_1SCl$ (III)

angelagert wird, worin $R_1$ einen gegebenenfalls substituierten Alkyl- oder Phenylrest bedeutet. Anschließend wird in der erhaltenen Verbindung der Formel

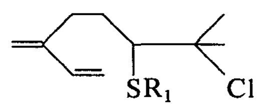

(IV)

das Chlor in üblicher Weise durch den Rest $OR_2$ ersetzt, wobei $R_2$ für einen Acylrest, einen Sulfonsäurerest, einen Tetrahydropyranyl- oder Methoxytetrahydropyranylrest, einen Triarylmethyl- oder einen Silylrest steht, sodann mit üblichen Oxidationsmitteln in der Verbindung der Formel

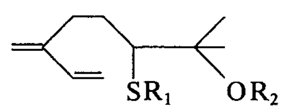

(V)

den Schwefel oxidiert, wobei eine Verbindung der Formel

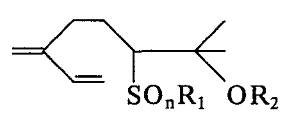

(VI)

entsteht, in der n 1 oder 2 bedeutet, während $R_1$ und $R_2$ die obige Bedeutung haben. Schließlich wird nach üblichen Methoden die Verbindung $R_1SO_2H$ eliminiert und aus der resultierenden Verbindung der Formel

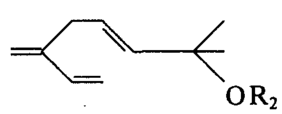

(VII)

die Gruppe $R_2$ unter Austausch gegen Wasserstoff mit sauren, basischen oder neutralen Agenzien abgespalten.

$R_2$ hat den Charakter einer Schutzgruppe, die je nach ihrer Art mit sauren, basischen oder neutralen Agenzien abgespalten wird. Geeignet sind besonders gegebenenfalls substituierte Acylreste, z. B. Formyl, Acetyl, Trifluoracetyl, Propionyl, Benzoyl, 4-Nitrobenzoyl, Reste anderer Säuren, z. B. von Sulfonsäuren, etwa Tosyl. Diese Reste werden im allgemeinen mit basischen Mitteln entfernt, beispielsweise mit Alkoholaten, etwa Natriumalkoholat in dem betreffenden Alkohol, oder mit Kaliumhydrogencarbonat/wäßrigem Methanol im Falle des Formylrestes.

Der Schutz der Hydroxygruppen kann auch durch eine Acetal- oder Ketalgruppierung erfolgen, wobei $R_2$ beispielsweise einen gegebenenfalls substituierten Tetrahydropyranyl- oder Methoxytetrahydropyranylrest

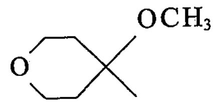

darstellt.

Diese Gruppen sowie die ebenfalls geeigneten Triarylgruppen, z. B. Trityl, Mono-, Di- oder Trimethoxytrityl, werden durch Säureeinwirkung entfernt.

Auch Silylgruppen eignen sich gut als Schutzgruppen, etwa Trimethylsilyl, das durch Erhitzen mit wäßrigem Alkohol abgespalten werden kann, ferner beispielsweise Silylgruppen der Formeln $Si(CH_3)_2C(CH_3)_3$ oder $Si(CH_3)_2C_6H_5$. Zur Abspaltung dieser Gruppen dient Kaliumfluorid in einem geeigneten Lösungsmittel.

Die Anlagerung des Sulfenylchlorids, beispielsweise der Phenylverbindung, an die Doppelbindung von Myrcene erfolgt bei niederer Temperatur. Im Reaktionsprodukt wird, gegebenenfalls ohne vorherige Reinigung, Cl durch $R_2O$ ersetzt. Für $R_2$ = Acyl kann dies durch Umsetzung mit einem Salz der entsprechenden Carbonsäure, z. B. Natriumacetat, geschehen. Nach Reinigung durch Säulenchromatographie erhält man Verbindung V. Die Strukturzuordnung gelingt durch spektroskopische Daten, insbesondere NMR.

Für die Oxidation von V zu VI bedient man sich der Methoden, die für die Oxidation eines Thioäthers zu einem Sulfoxid gebräuchlich sind. Geeignete Oxidationsmittel sind demnach beispielsweise Wasserstoffperoxid, Benzoepersäuren, Sauerstoff bzw. Luft plus Katalysatoren. Je nach den Oxidationsbedingungen entsteht neben dem Sulfoxid auch das entsprechende Sulfon.

Die Eliminierung von $R_1SOH$ bzw. $R_1SO_2H$ erfolgt beim Erhitzen von VI mit einer geeigneten basischen Substanz, z. B. einer organischen Base, wie Pyridin, Triäthylamin, oder mit einem Alkalihydrogencarbonat, z. B. Natriumhydrogencarbonat. Als Reaktionsmedium dienen gewünschtenfalls inerte organische Lösungsmittel, z. B. aromatische oder aliphatische Kohlenwasserstoffe, chlorierte Kohlenwasserstoffe, Äther.

Das Amitinol kann zum Anlocken von Borkenkäfern, insbesondere der Gattung Ips amitinus (Eichh.), benutzt und damit zur leichteren Bekämpfung dieser Schädlinge verwendet werden.

In dem nachstehenden Beispiel ist das erfindungsgemäße Verfahren näher erläutert.

IR-Spektren wurden mit Perkin Elmer — PE 257 Spektrometer gemessen. NMR-Spektren wurden mit einem Brucker WH 90 (90 MHz) Gerät gemessen.

Normalerweise wurden 3—4%ige Lösungen in $CDCl_3$ verwendet, und $Si(CH_3)_4$ (=0 ppm) diente als Standard. Die Spektren sind in ppm und die Kopplungskonstanten in c/s (cycles per second) angegeben, während die Multiplizitäten wie folgt abgekürzt werden:

s = Singlet, d = Doublet, t = Triplet, M = Multiplet, br = breit.

Zuordnungen sind in Klammern gesetzt. Massenspektren (MS) wurden mit einem VARIANMAT-CH-7 Instrument bestimmt.
Elektronenenergie: 70 eV.

## Beispiel

1) Es werden 11,74 ml (0,069 Mol) Myrcene in 50 ml Methylenchlorid gelöst und die Mischung im Eisbad abgekühlt. Zu der gekühlten Lösung tropft man unter Rühren 10 g (0,069 Mol) Phenylsulfenylchlorid innerhalb von 15 Minuten hinzu. Man läßt 10 Minuten nachreagieren und engt am Rotationsverdampfer im Vakuum ein. Man erhält 19,2 g (99% d. Th.) orangefarbenes Öl. Es wird rasch weiterverarbeitet.
Charakterisierung: Massenspektrum (m/e) M+: 280/282

$M^+$ —Cl: 245; $M^+$ 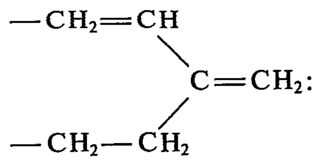 : 199/201

2) Man gibt 15,4 g (0,055 Mol) des nach 1) erhaltenen Rohprodukts und 9,02 g (0,11 Mol) Natriumacetat in 50 ml Essigsäure und rührt über Nacht bei Raumtemperatur. Anschließend gießt man auf Eiswasser und extrahiert die Suspension mit Chloroform. Die Chloroformphase wird abgetrennt, mit wäßriger Natriumhydrogencarbonatlösung und anschließend mit Wasser extrahiert. Die organische Phase wird mit $Na_2SO_4$ getrocknet und im Vakuum eingeengt.
Zur weiteren Reinigung des Produkts empfiehlt sich Säulenchromatographie an Kieselgel (Laufmittel: ein Gemisch aus Diisopropylether und Benzin). Man erhält 8 g eines gelb gefärbten Öls.

1 H-NMR: 1,37 (3 H, s); 1,53 (3 H, s); 1,79 (3 H, s);
1,27–2,94 (4 H, m) 3,82 (1 H, dd, $J_1$ = 11 Hz;
$J_2$ = 2,5 Hz); 4,86–5,38 (4 H, m); 6,33 (1 H, dd, $J_1$ = 17 Hz;
$J_2$ = 10,5 Hz) 7,01–7,61 (5 H, m)
JR = $\nu$max 1730 $cm^{-1}$ (s)

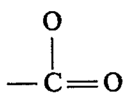

1635 $cm^{-1}$ (w)
C=C-Aryl; 1598 $cm^{-1}$ (m) C=C-Alken; 1240 $cm^{-1}$ (s) –C–O–;
742 $cm^{-1}$ (s) 5 H Aryl;

3) 4,7 g (0,0154 Mol) 2-Acetoxy-2-methyl-3-phenylthio-6-methylenoct-7-en werden mit 1,14 ml (0,0154 Mol) 30%igem $H_2O_2$ in 40 ml Eisessig für 4 Stunden bei 40° C gerührt. Anschließend wird auf Wasser gegossen und mit Chloroform extrahiert. Die organische Phase wird zuerst mit gesättigter wäßriger Natriumhydrogencarbonat-Lösung, dann mit Wasser geschüttelt. Nach dem Trocknen mit Natriumsulfat wird im Vakuum eingeengt.
Man erhält 2,6 g 2-Acetoxy-2-methyl-3-phenylsulfoxi-6-methylenoct-7-en als gelb gefärbtes Öl.
1H-NMR: 1.58 (3H, s); 1.69 (3H, s); 2.07 (3H, s); 6.15 (1H, dd $J_1$ = 11 Hz $J_2$ = 18 Hz); 7.3–7.71 (5H, m, Phenyl).

4) 2.6 g (0,0081 Mol) des gemäß 3) erhaltenen Öls und 1 g Natriumhydrogencarbonat werden in 30 ml Toluol zusammengegeben und das Gemisch 5 Stunden unter Rückfluß erhitzt.
Das überschüssige Natriumhydrogencarbonat wird abfiltriert und das Filtrat eingeengt. Der Rückstand enthält E-2-Acetoxy-2-methyl-6-methylenocta-3,7-dien und Sulfensäure bzw. deren Umwandlungsprodukte.

5) Der Rückstand von Stufe 4) wird ohne weitere Reinigung in 20 ml Methanol gegeben, in dem vorher 0,3 g Natrium gelöst wurden. Nach Rühren bei Raumtemperatur über Nacht wird im Vakuum das Lösungsmittel Methanol abdestilliert. Der Rückstand wird mit Ethylacetat digeriert und sodann mit wäßriger gesättigter Natriumhydrogencarbonat-Lösung und mit Wasser gewaschen. Die organische Phase wird im Vakuum eingeengt und der Rückstand durch Säulenchromatographie (Kieselgel, Laufmittel: Diisopropylether) gereinigt. Man erhält 0,3 g Amitinol als gelb gefärbtes Öl, Sdp. $_{14,7}$ mbar, 88° C.
$n_D^{27}$: 1,4918
1H-NMR: 1.31 (6H, s); 1.38 (OH, s); 2.91 (2H, br, d, J = 4 Hz); 4.91–5.33 (4H, m); 5.56–5.73 (2H, m); 6.38 (1H, dd, $J_1$ = 18 Hz, $J_2$ = 10.5 Hz).

## Patentansprüche

1. Verfahren zur Herstellung von E-2-Methyl-6-methylen-3,7-octadien-2-ol, dadurch gekennzeichnet, daß man an Myrcene ein Sulfenylchlorid $R_1SCl$ anlagert, worin $R_1$ einen gegebenenfalls substituierten Alkyl- oder Phenylrest bedeutet, anschließend in der erhaltenen Verbindung der Formel

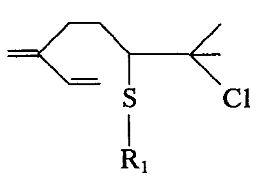

(IV)

das Chlor in üblicher Weise durch den Rest $OR_2$ ersetzt, wobei $R_2$ für einen Acylrest, einen Sulfonsäurerest, einen Tetrahydropyranyl- oder Methoxytetrahydropyranylrest, einen Triarylmethyl- oder einen Silylrest steht, dann mit üblichen Oxidationsmitteln den Schwefel oxidiert, so daß eine Verbindung der Formel

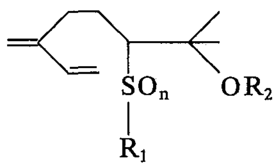

(VI)

(n = 1 oder 2) entsteht, hieraus $R_1SO_nH$ nach üblichen Methoden eliminiert und aus der resultierenden Verbindung der Formel

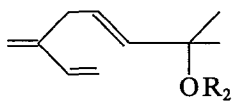

(VII)

die Gruppe $R_2$ unter Austausch gegen Wasserstoff mit sauren, basischen oder neutralen Agenzien abspaltet.

2. Verfahren nach Anspruch 1, dadurch gekennzeichnet, daß man in der letzten Stufe aus einer Verbindung der Formel VII, in der $R_2$ einen Acylrest oder Sulfonsäurerest bedeutet, die Spaltung mit einem Alkoholat durchführt.

**Claims**

1. Process for the preparation of E-2-methyl-6-methylene-3,7-octadien-2-ol, characterised in that a sulphenyl chloride $R_1SCl$ wherein $R_1$ represents an optionally substituted alkyl or phenyl group is added to myrcene, and then in the resulting compound of formula

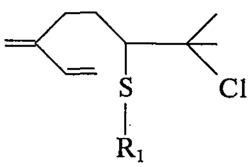

(IV)

the chlorine is replaced in the usual way by the group $OR_2$ wherein $R_2$ represents an acyl group, a sulphonic acid group, a tetrahydropyranyl or methoxy-tetrahydropyranyl group, a triarylmethyl or a silyl group, then the sulphur is oxidised with conventional oxidising agents so as to produce a compound of general formula

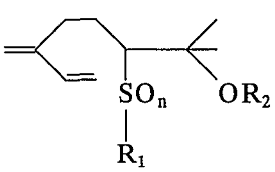

(VI)

(wherein n = 1 or 2), from which $R_1SO_nH$ is eliminated by conventional methods and the group $R_2$ is split off from the resulting compound of formula

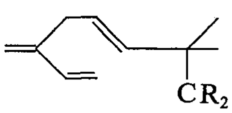

(VII)

in exchange for hydrogen, using acidic, basis or netral agents.

2. Process as claimed in claim 1, characterised in that, in the last stage, the splitting off from a compound of formula VII wherein $R_2$ represents an acyl group or a sulphonic aced group is effected with an alkoxide.

**Revendications**

1. Procédé de préparation du E-2-méthyl-6-méthylène-3,7-octadiène-2-ol, caractérisé en ce que l'on fixe sur le myrcène un chlorure de sulfényle $R_1SCl$, où $R_1$ représente un radical alcoyle ou phényle éventuellement substitué, on remplace ensuite dans le composé obtenu de formule

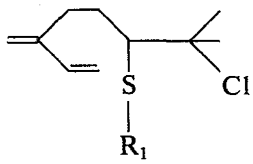

(IV)

le chlore, de manière habituelle, par le radical $OR_2$ où $R_2$ représente un radical acyle, un radical d'acide sulfonique, un radical tétrahydropyrannyle ou méthoxytétrahydropyrannyle, un radical triarylméthyle ou silyle, puis on oxyde le soufre avec des agents oxydants habituels, de sorte qu'il en résulte un composé de formule

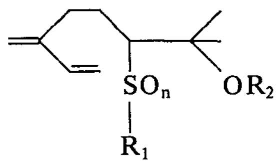

(VI)

(n = 1 ou 2), à partir duquel on élimine $R_1SO_nH$ selon des méthodes habituelles et on clive, à partir du composé résultant de formule

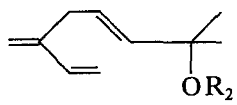

(VII)

le groupe $R_2$ par échange contre de l'hydrogène, au moyen d'agents acides, basiques ou neutres.

2. Procédé selon la revendication 1, caractérisé en ce que, dans la dernière étape, le clivage à partir d'un composé de formule VII dans laquelle $R_2$ représente un radical acyle ou un radical d'acide sulfonique est effectué au moyen d'un alcoolate.